# EUROPEAN PATENT APPLICATION

(11) **EP 2 597 611 A1**
(43) Date of publication of application: **29.05.2013**
(21) Application number: 12187099.2
(22) Date of filing: 03.10.2012
(51) Int. Cl.: G06Q 40/08, G06Q 50/22

(54) **System and method for analyzing audit risk of claims-based submissions for medicare advantage risk adjustment**

(30) Priority: 28.11.2011 US 201113305503
(71) Applicant: Censeo Health LLC, Dallas, TX 75244 (US)
(72) Inventor: McCallum, Jack, Fort Worth, TX Texas 76126 (US)
(74) Representative: Carpmaels & Ransford

(57) **Abstract**

Systems and methods have been developed that provide for the examination of data available on members of a Plan and based on the examined data, and on each reported ailment for a member, a determination is made as to the risk level such member presents to the Plan. This risk level is a measure of how likely it is that an assigned ailment identified from a claim will be found to be in error during an official CMS audit of member medical records for a particular year. The examination is made without necessarily reviewing actual physician's charts.

## Description

### CROSS-REFERENCE TO RELATED APPLICATIONS

The present application is related to commonly assigned U.S. Patent Application Serial No. 13/305,534 filed on November 28, 2011; entitled "SYSTEM AND METHOD FOR SCHEDULING HOME VISITS FOR PHYSICIAN REVIEW," the disclosure of which is hereby incorporated by referenced herein in its entirety.

### TECHNICAL FIELD

This disclosure relates to medical treatment audits and more particularly to systems and methods for determining risks associated with Medicare Advantage claims-based reimbursements.

### BACKGROUND OF THE INVENTION

Medicare Advantage Plans (herein the Plan) are run by private insurance companies that accept the underwriting and marketing responsibilities for Medicare members. The payment methodology which the Centers for Medicare and Medicaid services, (CMS) has established, allows a Plan to be paid different amounts for different members of the Plan based on a set of identified diagnoses that those members might have. Thus, for Plan members who are not diagnosed with one of the several categories of "higher cost" ailments, a basic amount (let's call that amount A) is paid to the Plan by Medicare. For each Plan member who is diagnosed with a higher cost ailment, such as diabetes, renal failure, etc., additional amounts are added to the basic amount. In our example, assume that diabetes is an additional amount A and renal failure is an additional amount B.

Accordingly, a Plan having 1,000 members would submit an invoice to Medicare that, by way of example, would look as follows: 700 members at A, 150 members at B, 100 members at C and 50 members at B+C.

The Plan determines the ailments (A, B, C, etc.) of its members from the claims it pays to the members (or to the member's physician) based on diagnostic information received from member's physicians. Thus, the information submitted to CMS is based on diagnostic data from physicians, , from review of member records, or from direct evaluation of the member by providers qualified to submit risk adjustment data to CMS. Based on the report (the number of A's, B's C's, etc.), CMS reimburses the Plan a certain amount.

The problem arises in that the CMS requires the ailment category (A, B, C, etc.) to be supported by actual physician charts on a patient by patient basis. However, since physicians are not reimbursed on the basis of the diagnoses, there is a high probability that the diagnoses captured from claims are not supportable by chart records. Studies have suggested that 30 to 35% of the diagnoses that are included in a CMS claim are not supported upon an actual review of the charts.

This is serious, since if the reimbursement made for a Plan member is not supported properly the Plan is in violation and must return the overpayment. The overpayment may be extrapolated from a small sample to the entire payment to the Plan for a given calendar year. Because the only support for a diagnosis is a chart note, and since the chart note must be made contemporaneously with a patient's visit, it may not be possible to support a claim after the fact, even if the diagnosis is correct. Because the errors in submission are extrapolated from a small sample to an entire contract membership, the dollar amounts involved with CMS payments are quite significant and the potential for large at-risk paybacks from Plans are significant.

### BRIEF SUMMARY OF THE INVENTION

Systems and methods have been developed that provide for the examination of data available on members of a Plan and based on the examined data, and on each reported ailment for a member, a determination is made as to the risk level such member presents to the Plan. This risk level is a measure of how likely it is that an assigned ailment identified from a claim will be found to be in error during an official CMS audit of member medical records for a particular year. The examination is made without necessarily reviewing actual physician's charts.

The foregoing has outlined rather broadly the features and technical advantages of the present invention in order that the detailed description of the invention that follows may be better understood. Additional features and advantages of the invention will be described hereinafter which form the subject of the claims of the invention. It should be appreciated by those skilled in the art that the conception and specific embodiment disclosed may be readily utilized as a basis for modifying or designing other structures for carrying out the same purposes of the present invention. It should also be realized by those skilled in the art that such equivalent constructions do not depart from the spirit and scope of the invention as set forth in the appended claims. The novel features which are believed to be characteristic of the invention, both as to its organization and method of operation, together with further objects and advantages will be better understood from the following description when considered in connection with the accompanying figures. It is to be expressly understood, however, that each of the figures is provided for the purpose of illustration and description only and is not intended as a definition of the limits of the present invention.

### BRIEF DESCRIPTION OF THE DRAWINGS

For a more complete understanding of the present invention, reference is now made to the following descriptions taken in conjunction with the accompanying drawing, in which:

FIGURE 1 shows one embodiment of a system for claims code verification;

FIGURE 2 shows one example of a table of Plan member diagnosed elements as contained in a database;

FIGURE 3 shows one example of one such report based upon the ailments contained in a table of a databases;

FIGURE 4 shows one example of a table of ailment codes contained in a database;

FIGURE 5 shows one embodiment of a method for performing verifications of ailment categories; and

FIGURE 6 shows one embodiment of a method for mitigating the risk of being unable to support an audit.

### DETAILED DESCRIPTION OF THE INVENTION

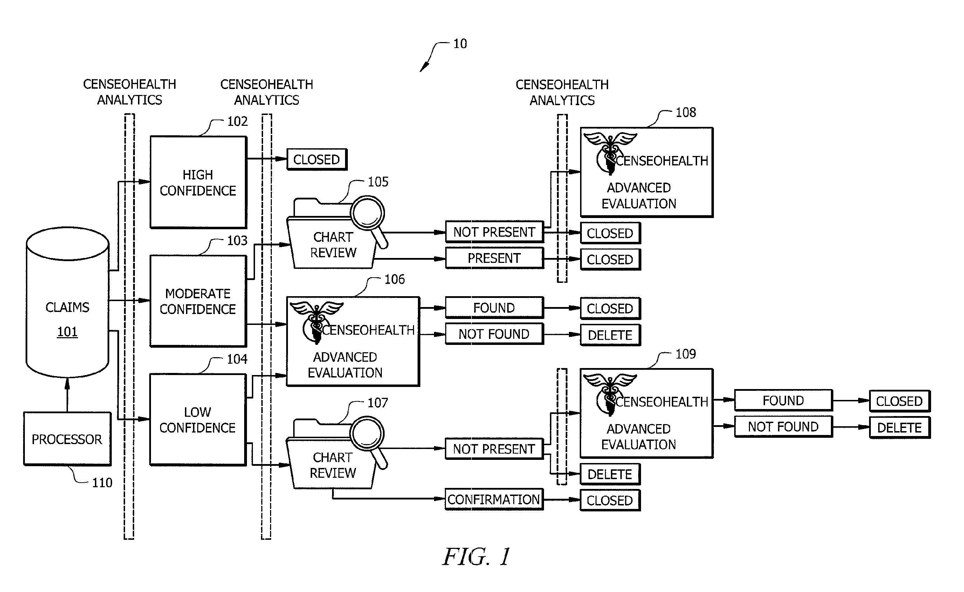

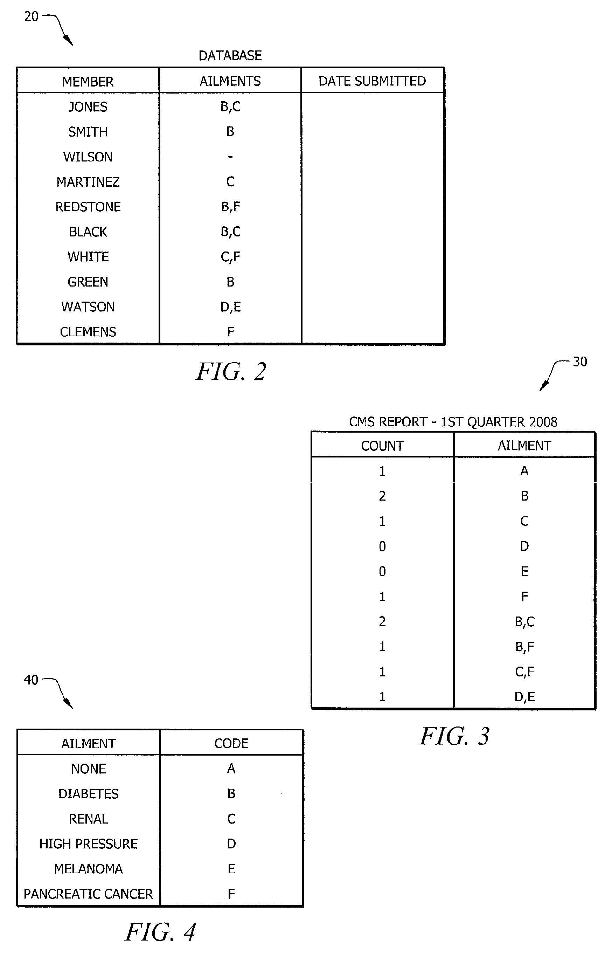

FIGURE 1 shows one embodiment 10 of a system for claims code verification. Claims that are made to CMS are stored, for example in database 101. An illustrative example of the database as it pertains to Plan members is shown in FIGURE 2, table 20. For example, member Jones has ailments B and C. From FIGURE 4, table 40 we know then that Jones has been diagnosed with diabetes and renal disease. Likewise, member Martinez has renal disease, while member Clemens is diagnosed with pancreatic cancer. As discussed above, these diagnosed ailments stem from diagnosis made by the member's attending physicians. At some point in time, perhaps quarterly, or monthly, reports are generated for presentation to CMS for the purpose of the Plan becoming reimbursed for money paid out to providers that attend to the medical needs of the Plan members. FIGURE 3 shows on example of one such report based upon the ailments contained in table 20 of database 101.

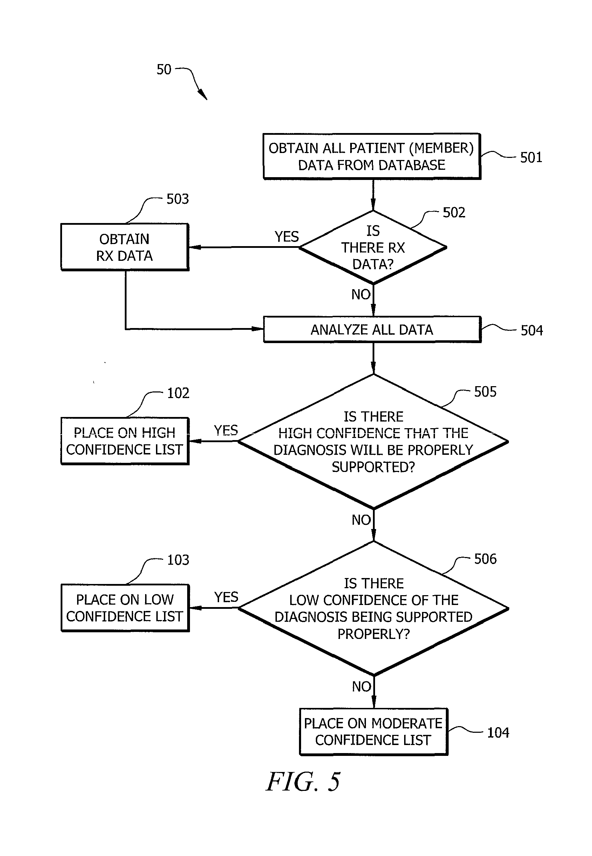

FIGURE 5 shows one embodiment 50 of a method for performing verifications of ailment categories. The methods used herein can be run under code control on a processor, such as processor 110 (FIGURE 1). When it is desired to validate the information contained in table 20 of database 101, process 501 obtains from database 101 (FIGURE 1) all of the records available for a given member or group of members. Note that this validation can occur before a report is filed with CMS or it can occur anytime in the same calendar year that the service was rendered. If performed in the same calendar year as the original service, then appropriate corrective steps, as discussed hereinafter, can be applied. If performed after a CMS filing, then the Plan can take remedial action by modifying the amount of funds being requested for reimbursement, or by paying back excess funds already reimbursed, or by seeking another source of verification of the diagnosis.

The data obtained from the database as shown in process 504, also contains pharmacy payments in many situations, as controlled by processes 502 and 503. The system also looks at the CMS Return Files (those files sent by CMS to the Plan that verify acceptance of submitted diagnoses with the implicit assumption that there is a supporting clinical record). The system examines the RAPS Submission File to obtain the set of diagnostic codes that were sent to CMS for a particular patient. The system also examines the RAPS Return File in which CMS acknowledges which of the diagnoses codes for a particular patient they have accepted as appropriate to the payment system. A file called the Model Output Report (MOR) is generated which is specific to each member. The MOR identifies the categories in which CMS has paid extra for diagnoses pertaining to a particular patient (member). The MMR file identifies the diagnoses that are attached to payments received by the Plan.

Process 504 then correlates all the data it can find, including the pharmacy data, for a given patient. This correlation is based on matching the diagnosed ailment(s) against other known data to determine if it all makes logical medical sense. For example, if the diagnosis is diabetes, the review of the file data should show medications consistent with diabetes. Also, the reviewed data showed that there is claim data from separate hospitalizations, each of which had a diagnosis of diabetes, the conclusion would be that there is a very high likelihood that a review of the member's actual chart (which typically resides with the attending physician and not in the Plan database) would support the diagnoses.

In this situation, process 305 would place that diagnosis for that patient in high confidence file 102 (shown also in FIGURE 1). The system then would count it as being very likely to be supportable for two reasons: one, is it came from multiple sources and two, it came from an institution. There is a difference between institutional and outpatient provider coding. The institutions get paid based on the diagnoses; the physician providers only get paid based on procedures that they perform. It is in the institution's interest that their diagnostic coding be accurate, so they hire professional coders and they tend to be accurate. If the system finds multiple institutional source claims-based submission, then that diagnosis for that patient is put in the high confidence category.

In similar fashion, if process 505 finds medications prescribed over a period of time consistent with the diagnosis of diabetes, or if process 505 finds that more than one physician has performed treatments or prescribed medications consistent with diabetes, the diagnosis for this patient is also put on the high confidence list. In some situations, the audit of the member's file might show that an actual chart has already been reviewed and is consistent with the claim. This also is a high confidence reimbursement. Another high confidence claim is one in which a home visit by a physician (as discussed below) has occurred for some other purpose. In such a situation there is a high likelihood that a chart properly noting conditions that support a particular diagnosis is available to support a CMS audit.

If a diagnosis for a particular patient is not placed on the high confidence list, then process 506 examines data from the database to see if more than one physician has made the same diagnosis, or to see if medications over time are consistent with the diagnosis. Other correlations, such as use of procedures, studies, or medical devices that would support presence of a specific clinical condition or identification of outpatient providers known to code accurately or inaccurately could be used to rank confidence levels. For example, diagnosis can only be made on the basis of a face to face encounter between a physician qualified provider and the patient (member). If the available data does not yield a high probability that such an encounter has occurred during a calendar year, the diagnosis is flagged by process 506 as having a low confidence level. Such face to face encounters can be inferred from the actions taken and from the coding used by the physician for reimbursement. Also, if a physician is on the CMS "unqualified to submit claims list" then diagnosis from that physician are tagged as having low confidence.

If process 506 finds some data that could support a proper diagnosis, but not enough to say for certain (process 505) that there is a high probability of accuracy, but more than a clear low confidence decision, then that diagnosis for that client is placed on the moderate confidence list. In the moderate range would be, for instance, a diagnosis which was not present in the prior year, is only present in this calendar year, and only came from a single encounter with a single provider. It is possible that the diagnosis is correct and if pushed, a chart note could be produced, but there remains the possibility that there would not be a chart note to support it.

The system uses a set of logic rules that go through the data and ranks the claims-based submissions bases on their level of risk so that at the end the data review, the system can say that in the population examined for this calendar year (because this whole process runs on a calendar year basis and resets every January 1^{st}) there are this many on the high confidence list, this many on the low confidence list and this many on the moderate confidence list. Said another way, there are this may (low confidence list) that most likely are not supportable and this many who, with a bit of probing, will likely find to be supportable. The rest of the patient diagnosis are most likely not going to be a problem. Note that in the low probability list there can be rankings, such that some can be tagged as having no-or almost no- chance of being supported. Note also that a given patient may have multiple diagnoses, some of which might be highly supportable and some of which questionable. In some embodiments, the source of the claim is a main determinant of how safe it is.

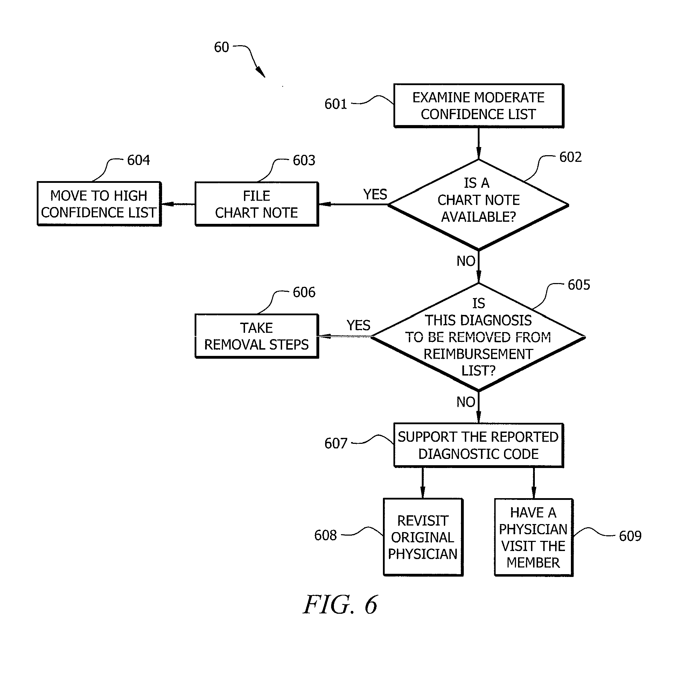

FIGURE 6 shows one embodiment of method 60 for mitigating the risk of being unable to support an audit. As shown in FIGURES 1 and 6, once the data is parsed into lists (there can be more than three lists if desired, depending on criteria used) process 601 begins the process the overall risk of overbilling (or even under billing in some situations). For the high confidence list, nothing more needs to be done.

A chart review can be conducted for the moderate confidence list by obtaining a copy (or physically examining the original) from the attending physician. The retrieved chart is then reviewed by a coder, as controlled by process 602. Then one of two things happens. One, the coder indicates that the chart is good and supports the note. In that situation, process 603 files the chart note so that it will be available to support an audit. Process 604 then places this diagnosis, for this patient, on the high confidence list.

If process 602 concludes that the chart does not support the note then process 605 can invoke process 606 to take the necessary remedial steps to delete the diagnostic code from the CMS report and give back the money. One reason to invoke process 606 is because something was found during process 506 (FIGURE 5) that indicates there is no chance-or a very low chance-of finding proper support.

Alternatively, process 605 can invoke process 607 to find another method to obtain a medical chart note that supports the original diagnostic code.

One method to obtain a supporting chart note is to have the patient go back to the original physician, process 608. This would occur, for example, in those situations where it is determined that the diagnosis is proper but the note is not correct. Another method would be to have a qualified provider visit the member directly at his or her home or another location. By invoking process 609, a physician is sent out to the member to re-evaluate that member. Since chart notes must be made in the same year that the diagnosis is made (at least under current regulations), physician updated charts, processes 608 and 609, are a viable option if the audit and the remedy are performed in the same year as the diagnosis.

Although the present invention and its advantages have been described in detail, it should be understood that various changes, substitutions and alterations can be made herein without departing from the spirit and scope of the invention as defined by the appended claims. Moreover, the scope of the present application is not intended to be limited to the particular embodiments of the process, machine, manufacture, composition of matter, means, methods and steps described in the specification. As one of ordinary skill in the art will readily appreciate from the disclosure of the present invention, processes, machines, manufacture, compositions of matter, means, methods, or steps, presently existing or later to be developed that perform substantially the same function or achieve substantially the same result as the corresponding embodiments described herein may be utilized according to the present invention. Accordingly, the appended claims are intended to include within their scope such processes, machines, manufacture, compositions of matter, means, methods, or steps.

## Claims

1. A computer-controlled method of minimizing risk of a non-supportable medical reimbursement, said reimbursement intended to compensate for expenses of a health group for medical diagnoses of said health group's members, said method comprising:
separating, under at least partial control of a processor, reimbursement requests pertaining to a particular group of said members into confidence levels; and
rehabilitating, under at least partial control of a processor, reimbursement requests that fall into a particular confidence level.

2. The method of claim 1 wherein said confidence levels are high, moderate and low, and wherein said rehabilitating is with respect to said low confidence level.

3. The method of claim 2 wherein said separating comprises:
reviewing, under at least partial control of said processor, each member's records associated with a reimbursement request based on a diagnosis for said member, said reviewing determining if at least one of the following exists in said reviewed records: a previously reviewed chart pertaining to said member, institutional billing containing a same diagnosis for said member, more than one provider coding said diagnosis, drug records matching said diagnosis, medical device use supporting a diagnosis, procedures that can be linked to a diagnosis, or related diagnoses that could support a diagnosis to arrive at said high confidence level in said diagnosis for said member.

4. The method of claim 2 wherein said separating comprises:
reviewing, under at least partial control of said processor, each member's records associated with a reimbursement request based on a diagnosis for said member, said reviewing determining if at least one of the following exists in said reviewed records: a single instance of coding for said diagnosis; contra-indicated diagnosis, indication of no face-to-face contact between said member and a provider, submission from a provider type not qualified to submit risk adjustment data, or submission from a provider known to document poorly to arrive at said low confidence level in said diagnosis for said member.

5. The method of claim 2 wherein said particular confidence level is said moderate confidence level, and wherein said rehabilitation comprises:
requesting, under at least partial control of said processor, each member to either i) visit said member's original diagnosis provider, or ii) be seen by a health care provider provided by said health group.

6. The method of claim 5 wherein said health group's health care provider has face-to-face contact with said member at a location identified by said member.

7. The method of claim 6 wherein said location is said member's place of residence and wherein a route followed by said health group's provider is established under control of said processor.

8. A processor for controlling an audit of medical reimbursements, said reimbursement intended to compensate for expenses of a health group for medical diagnoses of said health group's members, said processor operative to:
separate, in conjunction with human involvement, reimbursement requests pertaining to a particular group of said members into confidence levels; and
arrange, in conjunction with human involvement, for a confirming chart note for each member's diagnosis within a particular confidence level, if time permits, said confirming chart note pertaining to said member's diagnosis.

9. The processor of claim 8 wherein said confidence levels are high, moderate and low, and wherein said rehabilitating is with respect to said low confidence level.

10. The processor of claim 9 wherein said high confidence level is **characterized by** a determination that at least one of the following exists in a review of said member's records: a previously reviewed chart pertaining to said member, institutional billing containing a same diagnosis for said member, more than one provider coding said diagnosis, drug records matching said diagnosis, medical device use supporting a diagnosis, procedures that can be linked to a diagnosis, or related diagnoses that could support a diagnosis.

11. The processor of claim 9 wherein said low confidence level is **characterized by** a determination that at least one of the following exists in a review of said member's records: a single instance of coding for said diagnosis; contra-indicated diagnosis, indication of no face-to-face contact between said member and a provider, known suspect provider, submission from a provider type not qualified to submit risk adjustment data, or submission from a provider known to document poorly.

12. The processor of claim 9 wherein said particular level is said moderate confidence level, and wherein said rehabilitation comprises:
requesting each member to either i) visit said member's original diagnosis provider, or ii) be seen by a health care provider provided by said health group.

13. The processor of claim 12 wherein said health group's health care provider has face-to-face contact with said member at a location identified by said member.

14. The processor of claim 13 wherein said location is said member's place of residence and wherein a route followed by said health group's provider is established under control of said processor.
